# EUROPEAN PATENT SPECIFICATION

(11) **EP 0 619 731 B1**
(45) Date of publication and mention of the grant of the patent: **04.10.2000**
(21) Application number: 93900553.4
(22) Date of filing: 18.11.1992
(51) Int. Cl.: A61K 9/14, A61K 9/16

(54) **MICROCRYSTALLINE CELLULOSE SPHERONIZATION COMPOSITION**
ZUSAMMENSETZUNG AUF DER BASIS VON MIKROKRISTALLINER ZELLULOSE FÜR DIE HERSTELLUNG VON KUGELFÖRMIGEN TEILCHEN
COMPOSITION DE SPHERONISATION A BASE DE CELLULOSE MICROCRISTALLINE

(30) Priority: 30.12.1991 US 816027
(43) Date of publication of application: 19.10.1994
(73) Proprietor: FMC CORPORATION, Philadelphia, PA 19103 (US)
(72) Inventor: ERKOBONI, David Frank, Lawrenceville, NJ 08648 (US); FIORE, Scott Allan, Coopersburg, PA 18036 (US); WHEATLEY, Thomas Albert, Richboro, PA 18954 (US)
(74) Representative: McCall, John Douglas
(86) International application number: PCT/US92/09983
(87) International publication number: WO 93/12768

(56) References cited:
- EP-A- 0 231 026
- EP-A- 0 288 732
- GB-A- 1 010 477
- GB-A- 2 202 143
- US-A- 4 138 475
- US-A- 4 744 987
- DRUG DEVELOPMENT AND INDUSTRIAL PHARMACY, vol.17, no.9, 1991 pages 1143 - 1156 J.A.B. FUNCK ET AL 'binder effectiveness for beads with high drug levels'

## Description

This invention relates to spheronization compositions and solid pharmaceutical dosage forms based thereon. More particularly, the invention pertains to a microcrystalline cellulose:hydrocolloid spheronizing composition capable of yielding a high percentage of spheroids of uniform size and sphericity and containing highly elevated drug levels.

In the food and pharmaceutical arts, it has long been the practice to prepare and utilize particulate formulations in which the individual particles are of a spherical configuration. Such contoured particulates afford a number of advantages. The sphere has the lowest surface to volume ratio. Spheres pack evenly in containers for ease of accurately measuring quantities of particulate materials by volume. In the field of pharmaceuticals, the sphere is the ideal geometric solid for the application of film coatings to effect controlled release of drugs and/or medicinals.

A number of methods have been developed for producing spheronized pharmaceutical compositions. For instance, the preparation of spherical oral dosage forms in pill molds and pellet presses dates back many years. Since these early times, many improvements have been made in the technology.

In recent years, a method of producing spherical particulates that is attracting increased attention of the pharmaceutical community is that of spheronization. The essential features of this technique typically involve the steps of: forming a dry blend of powdered components; wetting the dry blend with a liquid, usually aqueous, in the presence of a binding agent to give a plastic-like mass or granulation; extruding the mass through a screen or dye to form a spaghetti extrudate; cutting the extrudate into short cylinders followed by a rounding stage in which the cylinders are rolled on a grooved surface into spheres. For more detailed information on the process reference is made to "A New Technique for the Production of Spherical Particles" by A. D. Reynolds in Manufacturing Chemist & Aerosol News, June 1970.

Much of the interest in spheronization as a means of producing spherical particulates, or spheroids as they are commonly known, comes in the wake of the ever growing use of multiparticulate controlled-release oral dosage forms. These pharmaceuticals consist of drug bearing spheroids, generally about 0.8 to 1.2 mm in diameter, contained in capsules or compressed into tablets. By using a combination of spheroids having different drug release characteristics, a dosage form can be tailored to provide the desired drug delivery system.

In carrying out the spheronization, microcrystalline cellulose is included in the formulation as a preferred carrier or matrix medium for the active components.

Microcrystalline cellulose (MCC) is a partially purified depolymerized form of cellulose and is obtained by treating pulps derived from fibrous plant material with mineral acid. The acid preferentially attacks the less ordered or amorphous regions of the cellulose polymer chain, thereby exposing and freeing the crystalline sites which form cellulose crystallite aggregates. The reaction mixture is washed to remove the degraded by-products, the resulting wet-cake freed of water and the dried cellulose crystallite aggregates, or more commonly microcrystalline cellulose, recovered.

Microcrystalline cellulose is a white, odorless,tasteless, relatively free-flowing powder, insoluble in water, organic solvents, dilute alkalies and dilute acids. It finds wide use as a pharmaceutical excipient, being particularly effective as a binder in the manufacture of tablets by direct compression.

In formulating a drug delivery system in which the drug composition is composed of spherical particulates, certain properties for the spheroids are desirable. These include low friability, uniformity of size and sphericity or roundness and surface smoothness. Such characteristics ensure that on being coated, a film layer of even thickness will be deposited on the spheroids for precise rate of drug release.

Drug bearing spheroids, exhibiting the characteristics aforesaid to a considerable degree can be realized using microcrystalline cellulose as the excipient for the drug carrier and spheronizing agent. A small amount of other cellulose derivatives that absorb water (a hydratable cellulose) may be included in combination with the microcrystalline cellulose. Examples of such cellulosic derivatives are sodium carboxymethyl cellulose, hydroxy lower alkyl (C₁-C₆) cellulose, e.g., hydroxypropyl cellulose and hydroxypropylmethyl cellulose.

A typical spheroid preparation involves forming a dry blend of microcrystalline cellulose, a hydratable cellulose derivative and drug. Water is then added to the blend until a densely granulated mass is obtained. This is then passed through a cylinder (usually 1 mm) on an extruder adjusted to give a uniform, free flowing extrudate which is then spheronized. Representative descriptions of these formulations can be found in U.S. Patent Nos. 4,844,910; 4,867,985 and 4,867,987.

Although such formulations and procedures can yield a high percentage of even sized spheroids at low to moderate drug levels, size distribution of the spheroids begins to widen as drug levels exceed about 60%. As drug loadings are further increased above about 80%, the spheroids become even more varied in size and sphericity thereby diminishing their utility as components in controlled release formulations.

Spheroids exhibiting good uniformity of size and sphericity and containing elevated drug levels, upwards of 80%, can be produced by carrying out the spheronization process with a mixture of drug and a highly modified microcrystalline cellulose of the type described in U.S. Patent No. 3,539,365.

This cellulosic derivative is prepared by subjecting the washed filter cake containing about 40% microcrystalline solids from the acid degradation of cellulose, to intense attritive forces. The result is a further break up of the cellulose crystallite aggregates and an increase in submicron particles. As the attrition proceeds, a sufficient amount of sodium carboxymethyl cellulose is added to the aqueous mixture in order to coat the separated microcrystals. Upon completion of the attrition, the mixture is dried and recovered. The dried product is readily redispersible in aqueous media to give gels. It contains about 5% to 15% by weight of sodium carboxymethyl cellulose.

The material is made and sold by the FMC Corporation in several grades under the Avicel® RC and CL trademark. Its application in spheronization processes is disclosed in FMC Technical Bulletin "Avicel Spheres."

Although the attrited microcrystalline cellulose:-sodium carboxymethyl cellulose composition yields generally satisfactory spheroids, it forms a sticky granulation which clings to the spheronization equipment. In order to maintain spheroid output, the equipment must be periodically disassembled and cleaned. This detracts from the practicality of such microcrystalline cellulose composition for commercial scale production and use as a spheronizing agent.

The following documents are also known:
- Drug development and industrial Pharmacy, vol. 17, no.9, 1991, pages 1143-1156, J.A.B. Funck et al. "binder effectiveness for beads with high drug levels";
- GB-A-2 202 143 (Euroceltique SA).

A microcrystalline spheronizing composition has now been found that is surprisingly superior to the prior art compositions.

In accordance with the present invention, there is provided a composition of matter, useful in preparing spheroids by spheronization, characterized by particles of a spray dried aqueous slurry of non-attrited microcrystalline cellulose selected from the group consisting of non-attrited wet cake and non-attrited dried wet cake and a non-ionic hydrocolloid selected from the group consisting of methylcellulose and hydroxypropyl methylcellulose, the ratio of the micro crystalline cellulose component of said particles to the hydrocolloid component being in a weight ratio of from 99:1 to 70:30, the said components being in intimate association with one another.

The composition is especially useful in the production of spheroids having high drug loadings.

Also according to the present invention, there is provided a process for producing a particulate material, useful in the preparation of spheroids by spheronization, characterised by:
forming a well dispersed slurry of non-attrited microcrystalline cellulose selected from the group consisting of non-attrited wet cake and non-attrited dried wet cake in an aqueous solution of a non-ionic hydrocolloid selected from the group consisting of methylcellulose and hydroxypropyl methylcellulose, the amounts of microcrystalline cellulose and hydrocolloid components being such as to provide a component ratio within the range recited below for the particulate co-processed product, and
spray drying the aqueous slurry to yield a particulate co-processed product wherein the weight ratio of non-attrited microcrystalline cellulose to hydrocolloid is in the range of from 99:1 to 70:30.

The invention further includes a method of producing solid dosage forms based on the spheronizing composition, and the resulting solid dosage forms.

The particulate co-processed spheronizing composition of the invention contains as its two essential components, microcrystalline cellulose selected from non-attrited wet cake and non-attrited dried wet cake and a pharmaceutical grade, non-ionic hydrocolloid, selected from methylcellulose and hydroxypropyl methylcellulose. As understood herein, hydrocolloid means a hydrophilic resin or polymer. Although the percentage of these two components can vary considerably, the weight ratio of microcrystalline cellulose to hydrocolloid is desirably held to a range of from about 99:1 to about 70:30, or, when the hydrocolloid is methyl cellulose, preferably from about 97.5:2.5 to about 90:10 and most preferably from about 96:4 to 94:6.

The source of the microcrystalline cellulose is preferably wet cake from a conventional microcrystalline cellulose manufacturing process. Wet cake which has not yet been dried is sometimes referred to as "never dried" or hydrocellulose. The dried microcrystalline cellulose can be used but it must be vigorously agitated in aqueous media to reconstitute the cellulose crystallite aggregates.

An economic advantage of the spheronization compositions invention is that it can be manufactured without having to employ the more expensive highly attrited microcrystalline cellulose of the prior art composition. The microcrystalline cellulose component of the compositions of the invention, whether in the wet cake or dried stage is understood to be unattrited microcrystalline cellulose.

The concentration of the non-ionic hydrocolloid solution should be sufficient to permit interaction of the microcrystalline cellulose with the hydrocolloid. Such interaction is believed to involve adsorption of hydrocolloid molecules on the surface of the cellulose crystallite aggregates which constitutes the structure of nonattrited microcrystalline cellulose.

The affinity or attraction or other property of the hydrocolloid for the cellulose crystallites appears to vary from one member to another, as evidenced by differences in spheronization properties.

A hydrocolloid which has been found exceptionally effective for preparing the microcrystalline spheronization compositions of the invention is methyl cellulose. Granulations containing this hydrocolloid process very cleanly in the spheronization equipment with no evidence of sticking while giving a high percentage of spheroids having excellent uniformity of size distribution and sphericity.

In preparing the spheronizing compositions of the invention, microcrystalline cellulose is added to an aqueous solution of the non-ionic hydrocolloid and the mixture formed into a slurry under high energy shear. The microcrystalline cellulose is preferably wet cake formed in the conventional acid hydrolysis of cellulose pulps. Dried microcrystalline cellulose can also be used provided the high shear blending is sufficient to redisperse the cellulose crystallites.

Mixing of the microcrystalline cellulose and aqueous hydrocolloid is continued until interaction of the hydrocolloid with the cellulose crystallites is complete or has reached equilibrium. Normally, this takes about 10 to about 60 minutes. As previously noted, the hydrocolloid is believed to form an adsorbed layer on the cellulose crystallite surface.

The concentration of microcrystalline cellulose and hydrocolloid in the aqueous slurry is such that the weight ratios of these components in the dried solid will fall within the specified ranges of 99:1 to 70:30, microcrystalline cellulose:hydrocolloid. Generally speaking, total amounts by weight of slurry solids will vary from about 5% to about 25%.

Certain of the hydrocolloids may form viscous solutions or even gels in aqueous media making it difficult or impossible to produce a flowable slurry. This can usually be circumvented by employing a more dilute solution of the hydrocolloid; also increasing the temperature may increase fluidity. For example, methyl cellulose is available in various viscosities, ranging from about 0.01 to 0.4 N.s.m⁻² (10 to 400 centipoise) or higher in aqueous solution. When using the higher viscosity material, lesser concentrations can be employed.

After the blending is complete, the slurry is dried, preferably by spray drying. Conventional spray drying equipment and operating procedures are employed. Drying gas outlet temperature is ordinarily used to control residual moisture content of the co-processed particulate material. Moisture levels of about 0.5% to about 8.0% are satisfactory with preferred levels being about 3.0% to about 5.0%.

The particle size of the dried spheronizing composition approximates that of the microcrystalline cellulose, although some of the crystallite aggregates may form clumps of larger particles.

Spheroids are produced from the spheronizing microcrystalline cellulose compositions of the invention following known spheronization procedures, including extrusion spheronization and rotogranulation/rotoprocessing techniques. A dry blend of the composition and drug is first prepared. Water is then added slowly, with continuous mixing until a granulation of the requisite consistency is obtained. Alternatively, the drug, if it is water soluble, can be dissolved in the water, and this solution added to the MCC:hydrocolloid particulate composition. The amount of the drug to be added is fixed by the loadings to which the spheres are susceptible and may be predetermined by routine testing.

The wet granulation is extruded using suitably sized pierced screens and spheronized using a rotating disk having a ground surface. The spheres are then dried in a fluidized bed or other drying means to a suitable moisture level, typically about 0.5% to 5%.

As understood in the pharmaceutical art, the term "sphere" or "spheroid" means spherical particles having a diameter in the range of about 0.1 to 2.5 mm, more preferably from 0.5 to 2 mm and most preferably from 0.8 to 1.4 mm.

The co-processed microcrystalline cellulose:-hydrocolloid composition of the invention affords a surprisingly higher percentage of spheroids in the narrow 0.8-1.4 mm range than when the spheronization is carried out using a three component granulation of microcrystalline cellulose, hydrocolloid and drug as called for in the prior art; see cited U.S. Patent Nos. 4,844,910; 4,867,985 and 4,867,987 supra. For some reason not presently understood, the interaction or affinity between the microcrystalline cellulose and hydrocolloid is not evident in the aforesaid three component system.

Because of their excellent uniformity of size and sphericity, the spheroids produced with the spheronizing compositions of the invention can be evenly coated for precise controlled drug release.

A further advantage of the invention is that the granulation of the herein co-processed composition is less susceptible to overwetting than is normally the case. This "forgiving feature" means that fewer batches of unacceptable granulated material will be produced, even with less experienced operators.

Still another advantage is that the coprocessed compositions of the invention are not sticky, that is, do not stick to spheronization equipment, and therefore are adaptable to large, commercial scale, multi-batch spheronization operations.

The spheronizing compositions of the invention can be used to form spheres with virtually all pharmaceutical preparations and drugs, including combinations of drugs, and whether the drugs are water soluble or water insoluble. Typical of such drugs are the following:
o Analgesics - acetaminophen, ibuprofen, ketoprofen and the like, indomethacin, naproxen, acetaminophen with codeine and acetaminophen with propoxyphene napsylate.
o Antibiotics - erythromycin, cephalosporins, minocycline HCl.
o Antiepileptics - phensuximide, phenytoin sodium and valproate sodium.
o Antihistamines - chlorpheniramine maleate, diphenhydramine hydrochloride, triprolidine hydrochloride.
o Cough and Cold Drugs - dextromethorphan hydrobromide, ephedrine sulfate, guaifenesin, phenylpropanolamine hydrochloride, promethazine hydrochloride, and pseudoephedrine hydrochloride.
o Cardiovascular Drugs - captopril, chlorthiazide and hydrochlorthiazide, diltiazem, nadolol, papaverine hydrochloride, procainamide hydrochloride, propranolol hydrochloride, quinidine gluconate, quinidine sulfate.
o Gastrointestinal Drugs - cimetidine, loperamide hydrochloride and ranitidine.
o Respiratory Drugs - albuterol sulfate, aminophylline, theophylline.

The following examples illustrate the invention in greater detail.

### Example 1

### PREPARATION OF SPHERONIZING COMPOSITION

Methylcellulose (2.86 kg (6.3 lb) at 4% moisture) having a viscosity of 0.015 N.s.m⁻² (15 centipoise) (Dow Chemical Co. Methocel® A-15LV) was hydrated with 44.4 kg (97.9 lb) of water to form a solution containing 6% solids. The hydrocolloid was added slowly to the water with overhead stirring using a commercially available variable speed mixer. In a separate slurry tank, microcrystalline cellulose 124.6 kg (274.7 lb) of a 41.5% wet cake) was diluted with 130.5 kg (287.8 lb) of water. The microcrystalline cellulose was added to the water and dispersed using a high energy dispersator such as a Cowles mixer at 1500 RPM until a smooth cream-like slurry was formed. The methylcellulose solution was added to the microcrystalline cellulose with continued stirring for 40 minutes using the high energy mixer to form a uniform dispersion. The final slurry had a solids content of 18%. The slurry was then spray dried using a single fluid nozzle for atomization. The conditions used during the drying were 1500 psi (10.342 x 10³ KPa) line pressure and a flow rate of 0.5 gpm. An outlet temperature of 230°F-240°F (110°C-115.6°C) was maintained. The production rate at these conditions was 46 lbs/hr (20.87 kg/hr.).

### Example 2

A co-processed material containing 95% microcrystalline cellulose and 5% hydroxypropylmethyl cellulose was prepared using process and conditions similar to those described in Example 1.

### Example 3

### A. PREPARATION OF SPHEROIDS OF PRESENT INVENTION

The co-processed material described in Example 1 (95/5 microcrystalline cellulose/methylcellulose - MCC/MC) was used in the preparation of spheres containing 80% theophylline using the process commonly referred to as extrusion/spheronization. The co-processed 95/5 MCC/MC (200 g) along with 800 g of anhydrous theophylline was charged into a 12 quart planetary mixer and allowed to mix for a period of 5 minutes until a homogeneous mixture was achieved. To this mixture 600 g of water was added slowly with continued mixing for a period of 15 minutes allowing for sufficient distribution of the water throughout the mass. This process of wetting powders is commonly referred to as granulation. The so-obtained wet mass was then charged into the hopper of a Nica extruder. It was extruded through screens having 1.0 mm openings, at a speed of 14-16 RPM to form a pellet-like extrudate having an approximate length between 0.25 and 1.0 cm. The extrudate was then charged into the Nica spheronizer. This piece of equipment consists of a bowl having stationary side walls and a rotating bottom commonly referred to as the disk. The extrudate was processed in the spheronizer for a period of 8 minutes at a disk speed of 650 RPM. At the end of 8 minutes, the spheres were discharged into an appropriate receptacle. The spheres were then transferred onto trays of a conventional forced air oven and dried at 50°C for a period of 6 hours. 90% of the so obtained spheroids were within the range of 0.8 to 1.4 mm.

### B. PRIOR ART SPHEROIDS

Non-attrited microcrystalline cellulose (Avicel® PH-101, FMC Corporation, 190 g), methyl cellulose (10 g) and 800 g of anhydrous theophylline were dry blended and subjected to spheronization following the procedure of Example 3A. 73% of the so-obtained spheroids were within the size range of 0.8 to 1.4 mm. This Example illustrates the spheronizing procedure in which all three components aforesaid are spheronized as disclosed in cited U.S. Patent Nos. 4,867,985 and 4,867,987.

### C. PRIOR ART SPHEROIDS

Non-attrited microcrystalline cellulose (Avicel® PH-101, FMC Corporation, 200 g) and 800 g anhydrous theophylline were dry blended and subjected to spheronization following the procedure of Example 3A. 68% of the resulting spheroids were within the size range of 0.8 to 1.4 mm. This Example illustrates the spheronizing procedure in which microcrystalline is the sole spheronizing agent.

As is readily apparent from the foregoing comparative Example 3, the percent of spheroids in the desired range of 0.8 - 1.4 mm is surprisingly greater using the spheronizing composition of the invention (Example 3A) than is obtained with the prior art compositions (Examples 3B and 3C).

## Claims

1. A composition of matter, useful in preparing spheroids by spheronization, characterized by particles of a spray dried aqueous slurry of non-attrited micro-crystalline cellulose selected from the group consisting of non-attrited wet cake and nonattrited dried wet cake and a non-ionic hydrocolloid selected from the group consisting of methylcellulose and hydroxypropyl methylcellulose, the ratio of the micro crystalline cellulose component of said particles to the hydrocolloid component being in a weight ratio of from 99:1 to 70:30, the said components being in intimate association with one another.

2. A composition as claimed in claim 1, wherein the source of the microcrystalline cellulose component is never dried wet cake.

3. A composition as claimed in claim 1 or 2, wherein the hydrocolloid is methyl cellulose and the weight ratio of the two components is in the range of from 97.5:2.5 to 90:10 microcrystalline cellulose:hydrocolloid.

4. A composition as claimed in claim 1 or 2, wherein the hydrocolloid is methyl cellulose and the weight ratio of the two components is in the range of from 96:4 to 94:6 microcrystalline cellulose:hydrocolloid.

5. A composition as claimed in any one of claims 1 to 4, wherein the hydrocolloid is methyl cellulose.

6. A composition as claimed in claim 1, wherein the hydrocolloid component is hydroxypropylmethyl cellulose.

7. A process for producing a particulate material, useful in the preparation of spheroids by spheronization, characterised by:
forming a well dispersed slurry of non-attrited microcrystalline cellulose selected from the group consisting of non-attrited wet cake and non-attrited dried wet cake in an aqueous solution of a non-ionic hydrocolloid selected from the group consisting of methylcellulose and hydroxypropyl methylcellulose, the amounts of microcrystalline cellulose and hydrocolloid components being such as to provide a component ratio within the range recited below for the particulate co-processed product, and
spray drying the aqueous slurry to yield a particulate co-processed product wherein the weight ratio of non-attrited microcrystalline cellulose to hydrocolloid is in the range of from 99:1 to 70:30.

8. A process as claimed in claim 7, characterized in that the microcrystalline cellulose is never dried wet cake.

9. A process as claimed in claim 8, wherein the spray drying is carried out at a drier outlet temperature of from 110°C to 115.6°C (230°F to 240°F).

10. A method of producing a particulate spheronized solid dosage form characterized by subjecting a blend of a drug and a composition as claimed in any one of claims 1 to 6 to spheronization.

11. A solid dosage form characterized by spheres containing, in combination, a composition as claimed in any one of claims 1 to 6, and a pharmaceutically effective amount of at least one drug.

## Patentansprüche

1. Zusammensetzung eines Stoffes, der sich bei der Herstellung von Sphäroiden durch Sphäronisation als nützlich erweist, gekennzeichnet durch Teilchen eines sprühgetrockneten wäßrigen Breies aus nichtzerriebener mikrokristalliner Cellulose, die aus der Gruppe ausgewählt wird, die aus nichtzerriebenem feuchtem Kuchen und nichtzerriebenem getrocknetem feuchtem Kuchen und einem nichtionischen Hydrokolloid besteht, das aus einer Gruppe ausgewählt wird, die aus Methylcellulose und Hydroxypropyl-Methylcellulose besteht, wobei das Verhältnis des mikrokristallinen Cellulosebestandteils von genannten Teilchen zu dem Hydrokolloidbestandteil in einem Gewichtsverhältnis von 99:1 bis 70:30 vorliegt, wobei sich genannte Bestandteile in inniger Assoziation miteinander befinden.

2. Zusammensetzung nach Anspruch 1, worin die Quelle des mikrokristallinen Cellulosebestandteils niemals getrockneter feuchter Kuchen ist.

3. Zusammensetzung nach Anspruch 1 oder 2, worin das Hydrokolloid Methylcellulose ist und das Gewichtsverhältnis der beiden Bestandteile in dem Bereich von 97,5 : 2,5 bis 90:10 mikrokristalliner Cellulose : Hydrokolloid liegt.

4. Zusammensetzung nach Anspruch 1 oder 2, worin das Hydrokolloid Methylcellulose und das Gewichtsverhältnis der beiden Bestandteile in dem Bereich von 96 : 4 bis 94 : 6 mikrokristalliner Cellulose : Hydrokolloid liegt.

5. Zusammensetzung nach einem der Ansprüche 1 bis 4, worin das Hydrokolloid Methylcellulose ist.

6. Zusammensetzung nach Anspruch 1, worin der Hydrokolloidbestandteil Hydroxypropyl-Methylcellulose ist.

7. Verfahren zum Herstellen eines partikulären Materials, das sich bei der Herstellung von Sphäroiden durch Sphäronisation als nützlich erweist, gekennzeichnet durch:
Bilden eines gut dispergierten Breies aus nichtzerriebener mikrokristalliner Cellulose, die aus der Gruppe ausgewählt wird, die aus nichtzerriebenem feuchtem Kuchen und nichtzerriebenem getrocknetem feuchtem Kuchen in einer wäßrigen Lösung eines nichtionischen Hydrokolloids besteht, das aus der Gruppe ausgewählt wird, die aus Methylcellulose und Hydroxypropyl-Methylcellulose besteht, wobei die Mengen von mikrokristallinen Cellulose- und Hydrokolloidbestandteilen dergestalt sind, daß sie ein Bestandteil-Verhältnis in dem unten aufgezeigten Bereich für das partikuläre, miteinander verarbeitete Produkt vorsehen und
Spühtrocknen des wäßrigen Breies, um ein partikuläres, miteinander verarbeitetes Produkt zu ergeben, worin das Gewichtsverhältnis von nichtzerriebener mikrokristalliner Cellulose zu Hydrokolloid in dem Bereich von 99 : 1 bis 70 : 30 liegt.

8. Verfahren nach Anspruch 7, dadurch gekennzeichnet, daß die mikrokristalline Cellulose niemals getrockneter feuchter Kuchen ist.

9. Verfahren nach Anspruch 8, worin das Sprühtrocknen bei einer Trocknerauslaßtemperatur von 110°C bis 115,6°C (230°F bis 240°F) durchgeführt wird.

10. Verfahren zum Herstellen einer partikulären sphäronisierten festen Dosierungsform, dadurch gekennzeichnet, daß eine Mischung aus einem Arzneimittel und einer Zusammensetzung nach einem der Ansprüche 1 bis 6 der Sphäronisation unterzogen wird.

11. Feste Dosierungsform, dadurch gekennzeichnet, daß Sphären, in Kombination, eine Zusammensetzung nach einem der Ansprüche 1 bis 6 und eine pharmazeutisch wirksame Menge von mindestens einem Arzneimittel enthalten.

## Revendications

1. Composition de matière, utile dans la préparation de sphéroïdes par sphéronisation, caractérisée par des particules d'une boue aqueuse séchée par pulvérisation de cellulose microcristalline non détériorée par l'usage sélectionnée à partir du groupe consistant en gâteau humide non détérioré par l'usage et en gâteau humide séché non détérioré par l'usage et d'un hydrocolloïde non ionique sélectionné à partir du groupe consistant en méthylcellulose et en hydroxypropyl-méthylcellulose, le rapport du composant de cellulose microcristalline desdites particules au composant d'hydrocolloïde étant selon un rapport de pondération de 99:1 à 70:30, lesdits composants étant en association intime l'un avec l'autre.

2. Composition telle que revendiquée dans la revendication 1, dans laquelle la source du composant de cellulose microcristalline n'est jamais un gâteau humide séché.

3. Composition telle que revendiquée dans la revendication 1 ou 2, dans laquelle l'hydrocolloïde est de la méthylcellulose et le rapport de pondération des deux composants est dans la gamme de 97,5:2,5 à 90:10 de cellulose microcristalline:hydrocolloïde.

4. Composition telle que revendiquée dans la revendication 1 ou 2, dans laquelle l'hydrocolloïde est de la méthylcellulose et le rapport de pondération des deux composants est dans la gamme de 96:4 à 94:6 de cellulose microcristalline:hydrocolloïde.

5. Composition telle que revendiquée dans l'une quelconque des revendications 1 à 4, dans laquelle l'hydrocolloïde est de la méthylcellulose.

6. Composition telle que revendiquée dans la revendication 1, dans laquelle le composant d'hydrocolloïde est de l'hydroxypropyl-méthylcellulose.

7. Procédé de production d'une matière particulaire, utile dans la préparation de sphéroïdes par sphéronisation, caractérisé par:
la formation d'une boue bien dispersée de cellulose microcristalline non détériorée par l'usage sélectionnée à partir du groupe consistant en gâteau humide non détérioré par l'usage et en gâteau humide séché non détérioré par l'usage dans une solution aqueuse d'un hydrocolloïde non ionique sélectionné à partir du groupe consistant en méthylcellulose et en hydroxypropyl-méthylcellulose, les quantités des composants de cellulose microcristalline et d'hydrocolloïde étant telles à fournir un rapport de composants dans la gamme précisée ci-dessous pour le produit particulaire co-traité, et
le séchage par pulvérisation de la boue aqueuse pour donner un produit particulaire co-traité dans lequel le rapport de pondération de la cellulose microcristalline non détériorée par l'usage à l'hydrocolloïde est dans la gamme de 99:1 à 70:30.

8. Procédé tel que revendiqué dans la revendication 7, caractérisé en ce que la cellulose microcristalline n'est jamais un gâteau humide séché.

9. Procédé tel que revendiqué dans la revendication 8, dans lequel le séchage par pulvérisation est réalisé à une température de sortie de séchoir de 110°C à 115,6°C (230°F à 240°F).

10. Méthode de production d'une forme de dosage particulaire sphéronisée solide caractérisée par la soumission d'un mélange d'un médicament et d'une composition telle que revendiquée dans l'une quelconque des revendications 1 à 6 à la sphéronisation.

11. Forme de dosage solide caractérisée par des sphères contenant, en combinaison, une composition telle que revendiquée dans l'une quelconque des revendications 1 à 6, et une quantité pharmaceutiquement efficace d'au moins un médicament.
